# EUROPÄISCHE PATENTSCHRIFT

(11) **EP 2 248 464 B1**
(45) Veröffentlichungstag und Bekanntmachung des Hinweises auf die Patenterteilung: **16.07.2014**
(21) Anmeldenummer: 09159666.8
(22) Anmeldetag: 07.05.2009
(51) Int. Cl.: A61B 5/08

(54) **Die Nutzung des Anzahlstroms endogen generierter Partikeln in der Ausatemluft des Menschen zur Diagnose von Lungenkrankheiten**
Use of endogenous generated particles in the expired air of people to diagnose lung sicknesses
Utilisation du courant quantitatif de particules générées de manière endogène dans l'air d'expiration de l'homme pour le diagnostic de maladies des poumons

(43) Veröffentlichungstag der Anmeldung: 10.11.2010
(73) Patentinhaber: Fraunhofer-Gesellschaft zur Förderung der angewandten Forschung e.V., 80686 München (DE)
(72) Erfinder: Holfeld, Prof. Dr., Jens, 30629, Hannover (DE); Biller, Dr., Heike, 30625, Hannover (DE); Lödding, Hubert, 31275, Lehrte (DE); Dunkhorst, Wilhelm, 32469, Petershagen (DE); Schwarz, Katharina, 30519, Hannover (DE); Koch, Prof. Dr., Wolfgang, 31634, Steimbke (DE); Windt, Horst, 30938, Burgwedel (DE)
(74) Vertreter: Haggenmüller, Christian

(56) Entgegenhaltungen:
- DE-A1- 2 938 856
- DE-A1-102004 044 656
- US-A1- 2005 137 491
- KIM CHONG S ET AL: "Total lung deposition of ultrafine particles in elderly subjects during controlled breathing." INHALATION TOXICOLOGY 2005 JUN-JUL, Bd. 17, Nr. 7-8, Juni 2005 (2005-06), Seiten 387-399, XP009122807 ISSN: 0895-8378
- "Model 3025A Ultrafine Condensation Particle Counter" 1999, TSI INCORPORATED, , U.S.A , XP002546627 * das ganze Dokument *
- FAIRCHILD C I ET AL: "PARTICLE CONCENTRATION IN EXHALED BREATH" AMERICAN INDUSTRIAL HYGIENE ASSOCIATION JOURNAL, AKRON, OH, US, Bd. 48, Nr. 11, 1. Januar 1987 (1987-01-01), Seite 948/949, XP008030992
- 'Funktion_(Mathematik)', [Online] Wikipedia Deutsch Gefunden im Internet: <URL:http://de.wikipedia.org/wiki/Funktion_ (Mathematik)> [gefunden am 2011-08-10]

## Beschreibung

Die Erfindung betrifft ein Verfahren zur Analyse der Atemluft, welches im Rahmen der frühen, nicht-invasiven Diagnostik von krankhaften Veränderungen der peripheren Bereiche der Lunge (PAD, *peripheral airway disease*) wie sie z.B. bei obstruktiven Atemwegserkrankungen (Asthma bronchiale, chronisch obstruktive Lungenerkrankungen (COPD) oder bei der Bronchiolitits obliterans bzw. beim Bronchiolitis obliterans-Syndroms im Rahmen der Organabstoßung nach Lungentransplantation auftreten, Anwendung finden kann. Die Erfindung betrifft weiterhin eine Vorrichtung zur Analyse der Atemluft.

Durch die stetige Zunahme der Prävalenz von Lungenerkrankungen sind Früherkennung und Verlaufskontrolle zu einem herausragenden Ziel für die pneumologische Diagnostik geworden. Entscheidend hierbei ist eine möglichst spezifische und sensitive Methodik, die auf nicht-invasivem Wege Aussagen über die Lungenerkrankung erlaubt. Die nahe liegende Analytik der Ausatemluft hat in den letzten Jahren zunehmend Bedeutung erlangt. Nachdem vor zehn Jahren die grundsätzliche Messbarkeit nicht-volatiler Moleküle (Proteine, Peptide) in der menschlichen Ausatemluft gezeigt werden konnte, existieren mittlerweile eine Reihe deskriptiver Befunde zu Veränderungen der Konzentration verschiedener inflammatorischer Moleküle im Atemkondensat bei Patienten mit Lungenerkrankungen.

Nichtvolatile Moleküle müssen notwendigerweise über einen Tröpfchenbildungsprozess aus der Lungenflüssigkeit heraus in die Ausatemluft gelangen. Der exhalierte Anzahlstrom wird bestimmt zum einen durch die Rate der Tröpfchengeneration in den unterschiedlichen Lungenkompartimenten und zum anderen durch die Redeposition bereits generierter Partikeln in den komplexen Lungenstrukturen im Zuge des Exhalationsvorgangs. Daten aus der Literatur sowie aus eigenen Untersuchungen aus Vorexperimenten weisen auf ein Partikelgrößenspektrum mit einem Anzahlmaximum im Partikelgrößenbereich kleiner als 1 µm hin.

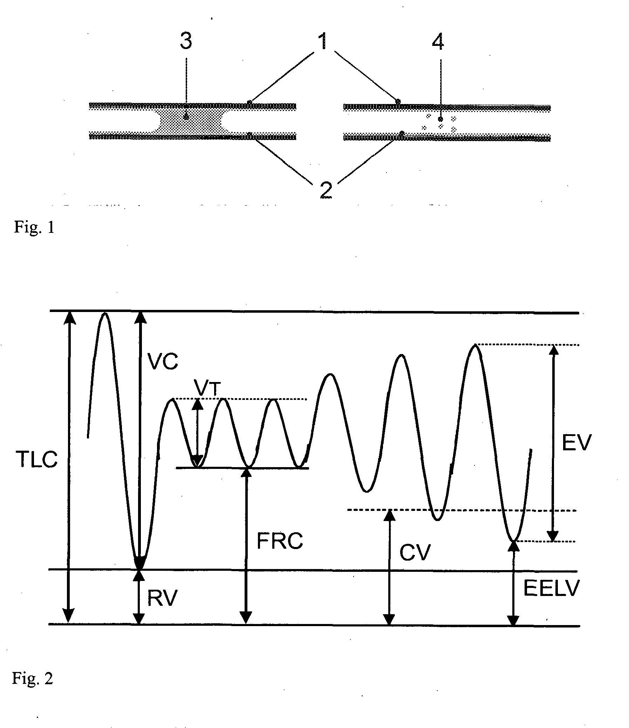

Ein Mechanismus der Tropfenentstehung in der Lunge ist die Wiederöffnung kollabierter Atemwegskapillaren der Lungenperipherie und die damit verbundene schlagartige Trennung des Epithelflüssigkeitsfilms, der die Atemwege auskleidet. Bei dieser flüssig-flüssig-Trennung entstehen Tröpfchen, die mit der Atemluft nach außen gelangen und detektiert werden können. Der Vorgang ist in Fig. 1 schematisch dargestellt, wobei die linke Ansicht eine verstopfte Lungenkapillare und die rechte Ansicht diese Kapillare nach der Wiedereröffnung zeigt. Oberflächenspannungsbedingte Instabilitäten (Rayleigh-Plateau-Instabilitäten) der Lungenflüssigkeit können zum Verschluss kleiner Atemwege führen. Flüssigkeitsbrücken bzw. Flüssigkeitspfropfen 3, wie in Fig. 1 gezeigt, stellen dabei einen energetisch günstigeren Zustand dar als die glatt mit Lungenflüssigkeitsfilm 2 ausgekleideten Kapillarwände 1. Dieser Zustand ist insbesondere beim Ausatmen realisiert. Beim Inspirationsvorgang steigt der transpulmonale Druck und es kann unter Ausbildung von Tröpfchen 4 zur Wiedereröffnung der beim Exspirationsvorgang verstopften Atemwege kommen.

Dieser Tropfenbildungsprozess ist von den Prozessen, die z.B. beim Sprechen oder Husten wirksam werden, zu unterscheiden. Letztere finden in den oberen Atemwegen statt und führen zu größeren, jedoch erheblich weniger Partikeln.

Der ventilierte Lungenbereich, in dem es zum Atemwegsverschluss kommen kann, wird als *closing volumen* (CV) bezeichnet. Wenn das Lungenvolumen am Ende eines Exspirationsvorgangs (EELV, *end expiratiory lung volume*) das *closing volume* unterschreitet (EELV<CV) kann es zur Realisierung von Atemwegsverschlüssen kommen. Die bei der Wiedereröffnung generierten Partikeln bzw. Flüssigkeitströpfchen werden dann beim nächsten Exhalationsvorgang mit dem Atemstrom nach außen getragen. Durch Variation der Atemtiefe ist die Verschlusswahrscheinlichkeit der peripheren Atemwege und mithin der Partikelstrom beeinflussbar. Dies steht jedoch in unmittelbarer Relation zu den individuell unterschiedlichen Lungenfunktionsparametern, insbesondere der Vitalkapazität und dem Residualvolumen, die in Fig. 2 dargestellt sind, welche, wie auch das *closing volume* für die gesunde Lunge eine Abhängigkeit vom Lebensalter aufweisen (siehe z.B. Milic-Emili, Eur. J. Appl. Physiol., 2007, 99: 567-583).

Fig. 2 zeigt übliche Lungenfunktionsparameter, die, außer dem Verschlussvolumen CV, mit gebräuchlichen Messverfahren wie Spirometrie oder Bodyplethysmographie bestimmt werden. Bei diesen in Fig. 2 gezeigten Lungenfunktionsparametern handelt es sich um die Lungenkapazität TLC ("total lung capacity"), das zur Atmung verfügbare Lungenvolumen VC ("vital capacity"), das nicht ventilierbare Residualvolumen RV ("residual volume"), das Atemzugvolumen in Ruheatmung VT ("tidal volume"), das Lungenvolumen bei Atemruhelage nach normaler Exspiration FRC ("functional residual capacity"), das Verschlussvolumen CV ("closing volume"), das Lungenvolumen am Ende des Exspirationsvorgangs EELV ("end expiratory lung volume") und das Ausatemvolumen EV ("expired volume").

Der Literatur ist ein Zusammenhang zwischen dem Verschlussvolumen und Veränderungen der peripheren Atemwege zu entnehmen. Schon in 1970er Jahren wurde postuliert, dass sich über die Messung des Verschlussvolumens eine Möglichkeit der frühen Diagnostik von Krankheiten der peripheren Atemwege eröffnet (siehe z.B. Buist und Ross, Chest, 1973, 63: 304-305). Des Weiteren wurde gezeigt, dass beim beatmeten Patienten, z.B. während einer Anästhesie, ein durch das Beatmungsmuster erzwungener periodisch wiederkehrender Atemwegsverschluss eine Verletzung der peripheren Atemwege hervorrufen kann (siehe z.B. Pelosi und Rocco, Critical Care, 2007, 11: 114-115).

Das Verschlussvolumen kann über die Gabe und Detektion von Edelgasboli im Rahmen von Lungenfunktionsuntersuchungen gemessen werden. Es wird beispielsweise Argon verwendet, dessen Konzentration in der Atemluft mit einem massenspektrometrischen Verfahren gemessen wird oder auch radioaktives ¹³³Xenon-Gas, verbunden mit einem szintigraphischen Detektionsverfahren.

Die Ereignisse der Wiedereröffnung der Atemwege können aber auch durch die Analyse akustischer Signale detektiert und quantifiziert werden. Sie äußeren sich in sog. Knistergeräuschen (*crackles*). Die Untersuchungen (siehe z.B. Alencar et al, Physica, 2005, A 357: 18-26) zeigen weiterhin, dass sich die Eröffnungsprozesse bei Überschreiten eines kritischen Druckes bei der Belüftung der explantierten Lunge lawinenartig verstärken. Prinzipiell eignet sich die Analyse der *crackles* für die frühzeitige Erkennung von peripheren Lungenveränderungen. Praktisch sind sowohl die Gasmessungen aufgrund der aufwendigen Detektion als auch die akustischen Messungen insbesondere wegen der Überlagerung mit anderen akustischen Signalen der Atmung sehr aufwendig.

DE 10 2004 044 656 A1 beschreibt ein Verfahren zum Messen der Partikelproduktion der menschlichen Lunge, welches das Ermitteln der Anzahl exhalierter Partikel, das Ermitteln des Exhalatvolumens und das Ermitteln der Partikelkonzentration umfasst.

Chong S.Kim et al., Inhalation Technology, 17: 3 87-399, 2005, beschreiben die Messung der Gesamtabscheidungsfraktion von ultrafeinen Partikeln in der Lunge.

Eine Aufgabe der vorliegenden Erfindung besteht in der Bereitstellung eines nicht invasiven Verfahrens zur Analyse der Atemluft, das einfach durchzuführen ist und als vorbereitende Maßnahme eine nachfolgende frühe Diagnostik von krankhaften Veränderungen der peripheren Bereiche der Lunge (PAD, *peripheral airway disease*) effizient unterstützt. Eine weitere Aufgabe der vorliegenden Erfindung besteht in der Bereitstellung einer Vorrichtung, mit der ein solches Verfahren möglichst effizient durchgeführt werden kann.

Gemäß eines ersten Aspekts der vorliegenden Erfindung wird die Aufgabe durch die Bereitstellung eines Verfahrens zur Analyse von Atemluft gelöst, welches folgende Schritte umfasst:
(a) optional die Messung mindestens eines Lungenfunktionsparameters LFP, welcher bevorzugt ausgewählt wird aus der Lungenkapazität TLC, dem zur Atmung verfügbaren Lungenvolumen VC, dem nicht ventilierbaren Residualvolumen RV, dem Atemzugvolumen in Ruheatmung VT, dem Lungenvolumen bei Atemruhelage nach normaler Exspiration FRC, dem Lungenvolumen am Ende des Exspirationsvorgangs EELV, oder Kombinationen davon, an einem Probanden P₁ durch Spirometrie und/oder Bodyplethysmographie,
(b) die gleichzeitige Messung des Atemflusses und der Konzentration Cₑ von Partikeln in der Ausatemluft für n unterschiedliche durchschnittliche Ausatemvolumina EVₓ, wobei n≥1 ist und x von 1 bis n läuft, an dem Probanden P₁,
(c) optional die Bestimmung der durchschnittlichen Anzahl Nₑ der Partikel in der Ausatemluft für jedes der unterschiedlichen durchschnittlichen Ausatemvolumina EVₓ,
(d) die Bestimmung einer Funktion Cₑ = f(EVₓ/A) und/oder einer Funktion Nₑ = f(EVₓ/A), wobei A entweder 1 oder ein Lungenfunktionsparameter LFP des optionalen Schritts (a) ist,
(e) die Bestimmung einer Partikelemissionskennzahl EKZ für den Probanden P₁ durch Auswahl eines definierten Funktionswertes oder Funktionsarguments der Funktion Cₑ = f(EVₓ/A) oder Nₑ = f(EVₓ/A), wobei A die unter Schritt (d) angegebene Bedeutung hat,
(f) die Wiederholung der Verfahrensschritte (a)-(d) an n Probanden Pₓ, wobei n≥1 ist und x von 2 bis n+1 läuft, und alle Probanden Pₓ sich untereinander und gegenüber P₁ durch mindestens einen biologisch-medizinischen Parameter BMP unterscheiden,
(g) die Bestimmung der Partikelemissionskennzahl EKZ für jeden Probanden Pₓ,
(h) die Bestimmung einer Funktion EKZ = f(BMP), d.h. die Bestimmung der Partikelemissionskennzahl EKZ als Funktion eines oder mehrerer biologisch-medizinischer Parameter BMP,
wobei die Messung der Konzentration von Partikeln in der Ausatemluft mit einem Kondensationskernzähler erfolgt, der keine Befeuchtungszone aufweist.

Das Verfahren beruht auf der Analyse der Anzahlkonzentration von Partikeln, die bei der Atmung in der Lunge generiert werden und im Exhalat gemessen werden können. Es wird hierbei einerseits ein Zusammenhang zwischen der Verschlusswahrscheinlichkeit peripherer Atemwege und abnormalen Lungenveränderungen als auch andererseits der Zusammenhang zwischen der Menge exhalierter Partikel und dem Wiedereröffnungsvorgängen verschlossener Atemwege ausgenutzt. Durch eine Kombination klinischer Lungenfunktionsmessungen und der Messung der Konzentration exhalierter Partikel bei unterschiedlichen Atemmustern kann somit im Rahmen einer dem erfindungsgemäßen Verfahren nachfolgenden Diagnose auf krankhafte Veränderungen in der Lunge geschlossenen werden. Im Rahmen der erfindungsgemäßen Verfahrens wird die Zahl der im Exhalat bzw. Ausatemvolumen emittierten Partikel gemessen, da erkannt wurde, dass diese Größe als möglicher Biomarker für krankhafte Veränderung der kleinen Atemwege genutzt werden kann.

Wie oben dargelegt, erfolgt in dem optionalen Schritt (a) des erfindungsgemäßen Verfahrens die Messung mindestens eines Lungenfunktionsparameters LFP, welcher bevorzugt ausgewählt wird aus der Lungenkapazität TLC, dem zur Atmung verfügbaren Lungenvolumen VC, dem nicht ventilierbaren Residualvolumen RV, dem Atemzugvolumen in Ruheatmung VT, dem Lungenvolumen bei Atemruhelage nach normaler Exspiration FRC, dem Lungenvolumen am Ende des Exspirationsvorgangs EELV, oder Kombinationen davon, an einem Probanden P₁ durch Spirometrie und/oder Bodyplethysmographie.

Die oben genannten Lungenfunktionsparameter sind dem Fachmann bekannt. Auch deren Messung durch Spirometrie und/oder Bodyplethysmographie ist dem Fachmann geläufig. In diesem Zusammenhang kann z.B. auf "Lungenfunktions-Manual", Wolfgang T. Ulmer, Thieme-Verlag, 2004, verwiesen werden.

Wird der Schritt (a) durchgeführt, so erfolgt die Spirometrie bevorzugt mit einem Ultraschallspirometer, einem Pneumotachographen, oder Kombinationen davon.

Wie oben dargelegt, erfolgt in Schritt (b) des erfindungsgemäßen Verfahrens die gleichzeitige Messung des Atemflusses und der Konzentration von Partikeln in der Ausatemluft für n unterschiedliche durchschnittliche Ausatemvolumina EVₓ, wobei n≥1, bevorzugt n≥2, bevorzugter n≥3 ist und x von 1 bis n läuft.

Bei der Messung des Atemflusses wird das inhalierte und exhalierte Atemvolumen in Abhängigkeit von der Zeit aufgezeichnet. Eine solche Messung des Atemflusses ist dem Fachmann bekannt, ebenso geeignete Messvorrichtungen für deren Durchführung.

Durch unterschiedliche Atemtiefe können durch den Probanden unterschiedliche Ausatemvolumina bzw. Exhalatvolumina EVₓ erzeugt werden. Wie nachfolgend noch eingehender diskutiert wird, erfolgt in einem späteren Schritt (d) die Bestimmung einer Funktion unter Verwendung von EVₓ/A (wobei A = 1 oder A= LFP ist) als Funktionsargument (d.h. "x-Wert"). Um eine Bestimmung der Funktion mit möglichst geringer Abweichung von den Messwerten zu ermöglichen, erfolgt die gleichzeitige Messung des Atemflusses und der Konzentration von Partikeln in der Ausatemluft in Schritt (b) bevorzugt für 4 Ausatemvolumina (d.h. EV₁ bis EV₄)

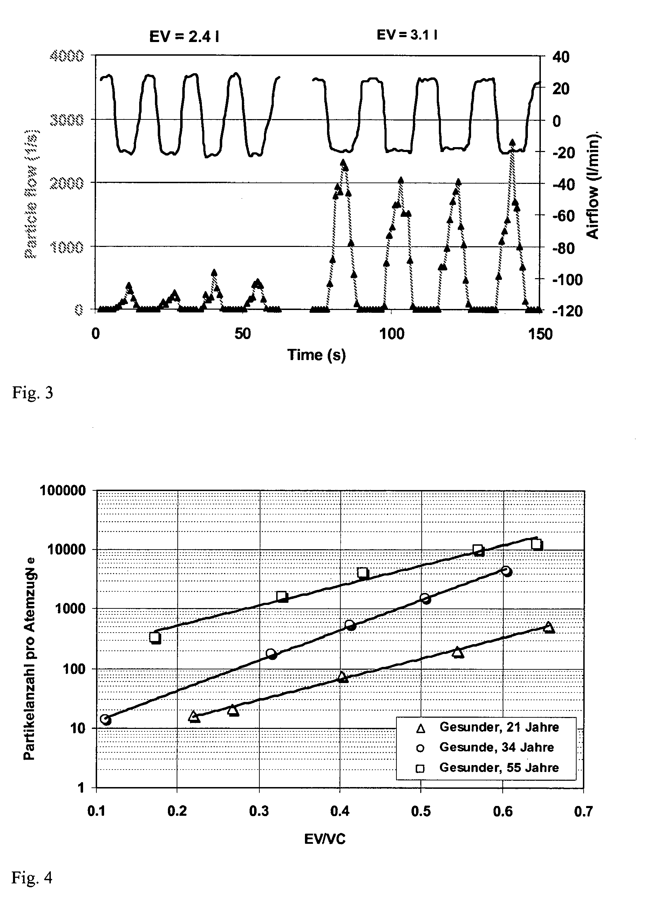

Eine beispielhafte gleichzeitige Messung des Atemflusses und der Konzentration von Partikeln in der Ausatemluft ist in Fig. 3 gezeigt. In der oberen Hälfte der Abbildung ist der Atemfluss (in 1/min) dargestellt, wobei in der ersten Minute die Atemtiefe geringer ist als in der zweiten Minute. Das durchschnittliche ausgeatmete Volumen bzw. eingeatmete Volumen ergibt sich als Mittelwert der Integrale über die jeweiligen Halbwellen der Volumenstromkurven (positive Halbwelle: Inhalation; negative Halbwelle: Exhalation). In dem in Fig. 3 gezeigten Messbeispiel ergibt sich für die erste Atemtiefe ein Ausatemvolumen von 2,4 1, während sich für die zweite Atemtiefe ein Ausatemvolumen von 3,1 ergibt. Die zeitgleiche Partikelmessung (siehe untere Hälfte der Fig. 3) ermöglicht die Bestimmung des Anzahlstroms von Partikeln in der ausgeatmeten Luft.

Bevorzugt erfolgt die Messung des Atemflusses für unterschiedliche durchschnittliche Ausatemvolumina EVₓ in Schritt (b) durch Spirometrie und/oder Bodyplethysmographie. Bevorzugt wird ein Pneumotachograph oder eine akustische Volumenstrommesseinrichtung, z.B. ein Ultraschallspirometer, verwendet.

Im Rahmen des erfindungsgemäßen Verfahrens ist es möglich, dass mit der Messung des Atemflusses in Schritt (b) auch die Messung des Lungenfunktionsparameters LFP gemäß Schritt (a) erfolgt.

Die Messung der Partikelkonzentration in der Ausatemluft kann mit solchen Messgeräten erfolgen, die auch noch Partikel mit sehr kleinen Durchmessern detektieren können. Bevorzugt sollte die Messvorrichtung für die Bestimmung der Partikelkonzentration in Schritt (b) eine untere Detektionsgrenze von 0,1 µm oder weniger aufweisen.

Der Begriff "Partikel" bezieht sich auf Flüssigkeitströpfchen, wie sie mit dem Exhalat beim Ausatmen ausgeschieden werden. Deren Entstehung in der Lunge wurde bereits oben unter Bezugnahme auf Fig. 1 diskutiert. Bevorzugt handelt es sich um Partikel (bzw. Flüssigkeitströpfchen oder flüssige Aerosolpartikel) mit einem durchschnittlichen Partikeldurchmesser von 2 µm oder weniger, noch bevorzugter 1 µm oder weniger.

Die Messung der Konzentration von Partikeln in der Ausatemluft in Schritt (b) erfolgt mit einem Kondensationskernzähler, der keine Befeuchtungszone aufweist.

Kondensationskernzähler sind dem Fachmann an sich bekannt und sind auch kommerziell erhältlich. Übliche Kondensationskernzähler haben eine Befeuchtungszone, in der sich ein Behälter mit Flüssigkeit befindet, sowie eine Kondensationszone, in der das Partikelwachstum stattfindet, indem der Dampf der Befeuchtungszone auf der Partikeloberfläche kondensiert.

Dieses Messverfahren mit Kondensationskernzähler erlaubt im Gegensatz zu reinen Streulichtpartikelzählern auch die Detektion von Partikeln mit Durchmessern kleiner als 0,1 µm. Partikel kleiner als 0,1 µm werden wegen ihres geringen Streuchlichtsignals mit reinen Streulichtzählern nicht mehr erfasst. Im Kondensationskernzähler lässt man die submikronen Partikeln durch Aufkondensieren eines übersättigtenDampfes (Butanol, Isopropanol, Wasser) auf Größen oberhalb von einigen Mikrometern anwachsen. Diese Partikeln können dann in einer einfachen Streulichtzelle gezählt werden. Aus der Zählrate, *R_{N}*, und dem Probenahmevolumenstrom ergibt sich die Konzentration, *C_{N}* der Partikel im Exhalat. Es ist vorteilhaft die Atemmanöver so durchzuführen, dass der Atemvolumenstrom immer größer ist als der Probenahmevolumenstrom. Dann ergibt sich die pro Atemzug emittierte Partikelanzahl durch einfaches Aufsummieren aller Zählereignisse während der Exhalationshalbwelle und Multiplikation mit dem Verhältnis aus Atemvolumen und Probenvolumen. Mit einem PC werden der Messvorgang gesteuert, die anfallenden Daten aufgezeichnet und die notwendigen Berechnungen durchgeführt. Er kann überdies über den Bildschirm dem Probanden visuell Vorgaben für die Durchführung des Atemmanövers geben.

Wie oben bereits erwähnt, sind Kondensationskernzähler kommerziell erhältlich. In vielen Geräten ist aber der Probenahmevolumenstrom mit 0,11/min so gering, dass bei den insgesamt niedrigen Emissionsraten die statistische Sicherheit für die Erfassung der Zählereignisse negativ beeinflusst werden kann. Weiterhin werden in den Geräten zum Teil gesundheitsschädliche Dämpfe (beispielsweise Butanol) verwendet, die anschließend aufwendig gefiltert werden müssen. Überdiese sind kommerzielle Geräte nicht zuletzt wegen ihres hohen technischen Aufwands zur Sättigung des Dampfes und einer anschließenden kontrollierten Abkühlung teuer.

Erfindungsgemäß weist der in Schritt (b) verwendete Kondensationskernzähler keine Befeuchtungszone (d.h. keinen Behälter mit Flüssigkeit) auf. Die Zunahme der Partikeldurchmesser im Kondensationskernzähler erfolgt im Wesentlichen, bevorzugter ausschließlich durch Kondensation des Wasserdampfes der Ausatemluft. Es wird also die Wasserdampfkondensation der schon mit Wasserdampf gesättigten Atemluft ausgenutzt.

Bevorzugt erfolgt in Schritt (b) die gleichzeitige Messung des Atemflusses durch Spirometrie und/oder Bodyplethysmographie und der Konzentration von Partikeln in der Ausatemluft mit einem Kondensationskernzähler in derselben Vorrichtung. Bevorzugt handelt es sich dabei um eine Vorrichtung, wie sie nachfolgend noch eingehender definiert wird.

Wie weiter unten noch diskutiert wird, werden die in Schritt (a), sofern durchgeführt, und/oder Schritt (b) erhaltenen Messdaten bevorzugt auf einem oder mehreren Datenspeichern abgelegt, um in nachfolgenden Schritten des erfindungsgemäßen Verfahrens bevorzugt einer oder mehreren Recheneinheiten zugeführt zu werden.

Wie oben dargelegt, erfolgt in einem optionalen Schritt (c) des erfindungsgemäßen Verfahrens die Bestimmung der durchschnittlichen Anzahl Nₑ der Partikel in der Ausatemluft für jedes der unterschiedlichen durchschnittlichen Ausatemvolumina EVₓ.

Die Anzahl Nₑ der Partikel in der Ausatemluft lässt sich z.B. für jedes der unterschiedlichen Ausatemvolumina EVₓ durch Multiplikation von Volumenstrom und Partikelkonzentration Cₑ bestimmen. In Fig. 3 erkennt man die deutliche Zunahme des Anzahlstroms (um ca. den Faktor 7) bei nur geringfügiger Zunahme der Atemtiefe um 25 %.

Wie oben dargelegt, erfolgt in Schritt (d) des erfindungsgemäßen Verfahrens die Bestimmung einer Funktion Cₑ = f(EVₓ/A) und/oder einer Funktion Nₑ = f(EVₓ/A), d.h. die Bestimmung der Konzentration Cₑ der Partikel in der Ausatemluft als Funktion des Verhältnisses EVₓ/A und/oder der Anzahl Nₑ der Partikel in der Ausatemluft als Funktion des Verhältnisses EVₓ/A, wobei A entweder 1 oder ein Lungenfunktionsparameter LFP des optionalen Schritts (a) ist.

Ist A ein Lungenfunktionsparameter (d.h. A = LFP), so handelt es sich bei diesem Lungenfunktionsparameter LFP bevorzugt um das zur Atmung verfügbare Lungenvolumen VC.

Bevorzugt handelt es sich bei der in Schritt (d) bestimmten Funktion Cₑ = f(EVₓ/A) bzw. Nₑ = f(EVₓ/A) um eine Exponentialfunktion.

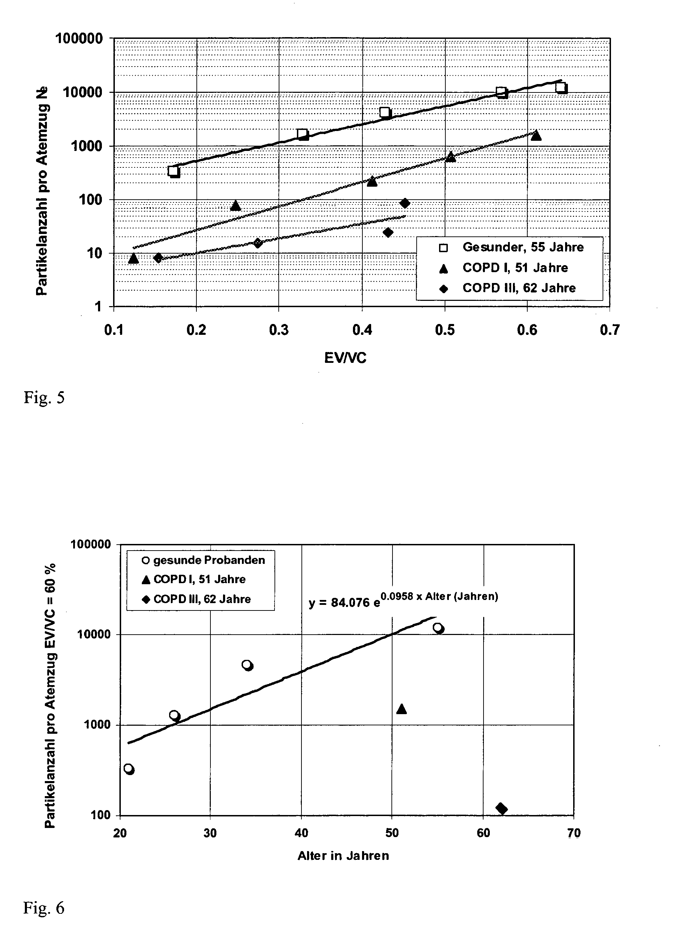

Eine beispielhafte Darstellung, in der die Abhängigkeit Nₑ = f (EV/VC) in einem Diagramm gezeigt wird, findet sich in den Abbildungen 4 und 5. An jeden Datensatz wurde eine Exponentialfunktion angepasst: Nₑ = B * exp(b*EV/VC). Diese Funktionen unterscheiden sich primär durch ihren Vorfaktor B und nicht so sehr durch den Wert des Exponenten, *b*.

Wie oben dargelegt, erfolgt in Schritt (e) des erfindungsgemäßen Verfahrens die Bestimmung einer Partikelemissionskennzahl EKZ für den Probanden P₁ durch Auswahl eines definierten Funktionswertes ("y-Wert") oder Funktionsarguments ("x-Wert") der Funktion Cₑ = f(EVₓ/A) oder Nₑ = f(EVₓ/A), wobei A die oben unter Schritt (d) angegebene Bedeutung hat.

Bevorzugt ergibt sich die Partikelemissionskennzahl EKZ aus dem Funktionswert ("y-Wert") der Funktion Cₑ = f(EVₓ/A) bzw. Nₑ = f(EVₓ/A) bei einem bestimmten, d.h. vorab festgelegten bzw. definierten Funktionsargument ("x-Wert") EVₓ/A, oder alternativ aus dem Funktionsargument EVₓ/A bei einem bestimmten, d.h. vorab festgelegten bzw. definierten Funktionswert der Funktion Cₑ = f(EVₓ/A) bzw. Nₑ = f(EVₓ/A). Der so ermittelte Funktionswert bzw. das so ermittelte Funktionsargument kann unmittelbar als Partikelemissionszahl EKZ verwendet werden oder kann alternativ einem weiteren Umrechnungsschritt unterworfen werden, der dann die Partikelemissionszahl EKZ des betreffenden Probanden liefert.

Beispielsweise kann es sich bei der Partikelemissionskennzahl EKZ in Schritt (e) um die Anzahl Nₑ bzw. die Konzentration Cₑ der Partikel bei EV/VC = 0.6 handeln.

Wie bereits oben ausgeführt, kann die Partikelemissionskennzahl EKZ als diejenige Anzahl an exhalierten Partikeln pro Atemzug bestimmt werden, bei der der Quotient, EV/VC, einen festen, vorgegebenen Wert annimmt, beispielsweise EV/VC = 60%. Andererseits kann aber auch der Quotient EV/VC bei vorgegebener exhalierter Partikelanzahl als Kennzahl gewählt werden. Das erste Verfahren ist beispielshaft für die Messdaten unterschiedlicher Probanden benutzt worden, und das Ergebnis ist in Fig. 6 dargestellt. Danach zeigt die Partikelemissionskennzahl eine eindeutige Abhängigkeit vom Alter (oder einem anderen geeigneten biologisch-medizinischem Parameter BMP).

Wie oben dargelegt, erfolgt im erfindungsgemäßen Verfahren nach Schritt (e) die Wiederholung der Verfahrensschritte (a)-(d) an n Probanden Pₓ, wobei n≥1 ist und x von 2 bis n+1 läuft, und alle Probanden Pₓ sich untereinander und gegenüber P₁ durch mindestens einen biologisch-medizinischen Parameter BMP unterscheiden. Im Rahmen der vorliegenden Erfindung wird unter einem "biologisch-medizinischen Parameter BMP" jede Eigenschaft eines Probanden verstanden, die geeignet ist, ihn in medizinischer oder biologischer Hinsicht von einem anderen Probanden zu unterscheiden.

Ein bevorzugter biologisch-medizinischer Parameter BMP im erfindungsgemäßen Verfahren kann das Alter des Probanden sein.

Wie nachfolgend noch eingehender diskutiert wird, erfolgt in einem späteren Schritt (h) die Bestimmung einer Funktion unter Verwendung eines oder mehrerer biologisch-medizinischer Parameter als Funktionsargument (d.h. "x-Wert"). Um eine Bestimmung der Funktion mit möglichst geringer Abweichung zu ermöglichen, sollten die Schritte (a) bis (d) an einer ausreichenden Zahl von geeigneten Probanden wiederholt werden, z.B. mindestens 3 oder sogar mindestens 4 Probanden.

Wie oben dargelegt, erfolgt in Schritt (g) des erfindungsgemäßen Verfahrens die Bestimmung der Partikelemissionskennzahl EKZ für jeden Probanden Pₓ. An dieser Stelle kann auf die Ausführungen über die Bestimmung der Partikelemissionskennzahl EKZ für Proband P₁ verwiesen werden.

Wie oben dargelegt, erfolgt in Schritt (h) des erfindungsgemäßen Verfahrens die Bestimmung einer Funktion EKZ = f(BMP), d.h. die Bestimmung der Partikelemissionskennzahl EKZ als Funktion eines oder mehrerer biologisch-medizinischer Parameter BMP.

Wie bereits oben diskutiert und in Fig. 6 beispielhaft dargestellt, zeigt die Partikelemissionskennzahl eine eindeutige Abhängigkeit von einem entsprechend ausgewählten, geeigneten biologisch-medizinischen Parameter wie z.B. dem Alter der Probanden.

Bevorzugt handelt es sich bei dem biologisch-medizinischen Parameter BMP in Schritt (h) um den gleichen biologisch-medizinischen Parameter BMP, wie er in Schritt (f) verwendet wurde.

Bevorzugt werden die Schritte (c)-(e), (g) und (h) jeweils unter Verwendung einer Recheneinheit durchgeführt werden. Bevorzugt handelt es sich um dieselbe Recheneinheit, die in allen Verfahrensschritten verwendet wird.

Bevorzugt werden die in den Schritten (a) und/oder (b) erhaltenen Messwerte auf einem oder mehreren Datenspeichern abgelegt und bevorzugt ist bzw. sind die oben erwähnte(n) Recheneinheit(en) programmtechnisch so eingerichtet, dass anhand der Messdaten die Konzentration Cₑ von Partikeln in der Ausatemluft ermittelt werden kann sowie die Bestimmung der durchschnittlichen Anzahl Nₑ der Partikel in der Ausatemluft in Schritt (c), die Bestimmung einer Funktion Cₑ = f(EVₓ/A) bzw. Nₑ = f(EVₓ/A) in Schritt (d), die Bestimmung einer Partikelemissionskennzahl EKZ in den Schritten (e) und (g), und/oder die Bestimmung einer Funktion EKZ = f(BMP) in Schritt (h) erfolgen kann bzw. erfolgt.

Bevorzugt wird das erfindungsgemäße Verfahren unter Verwendung der nachfolgend definierten Vorrichtung durchgeführt.

Gemäß eines weiteren Aspekts der vorliegenden Erfindung wird eine Vorrichtung zur Analyse von Atemluft bereitgestellt, umfassend:
- ein Rohr zur Aufnahme von Atemluft,
- zumindest ein Spirometer, und
- einen Kondensationskernzähler, wobei der Kondensationskernzähler keine Befeuchtungszone aufweist.

Bevorzugt weist das Rohr mindestens eine Abzweigung auf. Bevorzugt liegt das mindestens eine Abzweigung aufweisende Rohr als T-Stück oder X-Stück vor.

Bevorzugt weist das Rohr einen Inhalationszweig, der ausschließlich die Aufnahme von Atemluft aus der Umgebung ermöglicht, und einen Exhalationszweig, der ausschließlich die Abgabe von Atemluft an die Umgebung ermöglicht, aufweist. In einer bevorzugten Ausführungsform wird dies erreicht, indem sowohl im Inhalationszweig als auch im Exhalationszweig des Rohres ein Rückschlagventil angebracht ist.

Bevorzugt weist mindestens eine Abzweigung des Rohres ein Mundstück auf, durch das ein Proband ein- oder ausatmen kann.

Bezüglich des Spirometers kann auf die obigen Ausführungen zum erfindungsgemäßen Verfahren verwiesen werden. Bevorzugt wird das Spirometer aus einem Pneumotachographen, einem Ultraschallspirometer, oder Kombinationen davon ausgewählt.

In einer bevorzugten Variante kann sowohl im Inhalationszweig als auch im Exhalationszweig des Rohres ein Pneumotachograph angebracht sein. Zusätzlich oder alternativ kann in einer bevorzugten Ausführungsform in mindestens einer Abzweigung des Rohres, die bevorzugt weder der Inhalations- noch der Exhalationszweig ist, ein Ultraschallspirometer angebracht sein, welches sich bevorzugt unmittelbar vor dem Mundstück befindet.

Bevorzugt ist im Inhalationszweig des Rohres, bevorzugt auch im Exhalationszweig des Rohres, ein Filter, bevorzugt ein Absolutfilter angebracht.

Der Begriff "Absolutfilter" wird im Rahmen der vorliegenden Erfindung in seiner üblichen, dem Fachmann gebräuchlichen Bedeutung verwendet und bezieht sich auf einen Filter, der im Wesentlichen alle Partikel, mit einer Effizienz besser als 99,99 % abscheidet.

Mit einem Absolutfilter kann gewährleistet werden, dass die inhalierte Luft partikelfrei ist und somit die Partikelmessung am Exhalat nicht stört.

Im Inhalationszweig, in Strömungsrichtung der eingeatmeten Luft betrachtet, kann der Absolutfilter vor dem Rückschlagventil und optional vor dem Absolutfilter der Pneumotachograph angebracht sein, und im Exhalationszweig, in Strömungsrichtung der ausgeatmeten Luft betrachtet, kann das Rückschlagventil vor dem Absolutfilter und optional nach dem Absolutfilter der Pneumotachograph angebracht sein. Im Rahmen der vorliegenden Erfindung sind aber auch andere Anordnungen denkbar.

Wie bereits oben ausgeführt, weist der Kondensationskernzähler der erfindungsgemäßen Vorrichtung keine Befeuchtungszone, also keinen Bereich mit einem Behälter für die Aufnahme einer Flüssigkeit, die in der nachgeschaltenen Kondensationszone das Partikelwachstum bewirken würde, auf.

Bevorzugt weist der Kondensationskernzähler eine Düse mit in Strömungsrichtung konvergierendem Düsenquerschnitt für eine adiabatische Expansion auf.

Bevorzugt weist der Kondensationskernzähler eine Streulichtoptik für die Detektion von Partikeln auf, welche bevorzugt eine Sendeoptik zur Erzeugung eines Primärlichtstrahls und eine Empfangsoptik mit Lichtfalle beinhaltet.

Bevorzugt ist die Düse für die adiabatische Expansion in einem Düsenträger angebracht ist, der aus einem Material mit guter thermischer Leitfähigkeit gefertigt ist und optional mit einem Heizelement in thermischem Kontakt steht. Bevorzugt kann es sich bei dem Material mit guter thermischer Leitfähigkeit um Messing, Kupfer oder Kombinationen davon handeln.

Bevorzugt umfasst die erfindungsgemäße Vorrichtung weiterhin eine Recheneinheit und/oder einen Datenspeicher.

Bevorzugt handelt es sich bei der erfindungsgemäßen Vorrichtung um eine Vorrichtung zur nicht-invasiven Diagnostik von krankhaften Veränderungen der Lunge, insbesondere der peripheren Bereiche der Lunge (PAD, "peripheral airway disease") wie sie z.B. bei obstruktiven Atemwegserkrankungen (Asthma bronchiale, chronisch obstruktive Lungenerkrankungen (COPD) oder bei der Bronchiolitits obliterans bzw. beim Bronchiolitis obliterans-Syndroms im Rahmen der Organabstoßung nach Lungentransplantation auftreten.

Bevorzugt wird die erfindungsgemäße Vorrichtung zur Durchführung des oben beschriebenen Verfahrens verwendet.

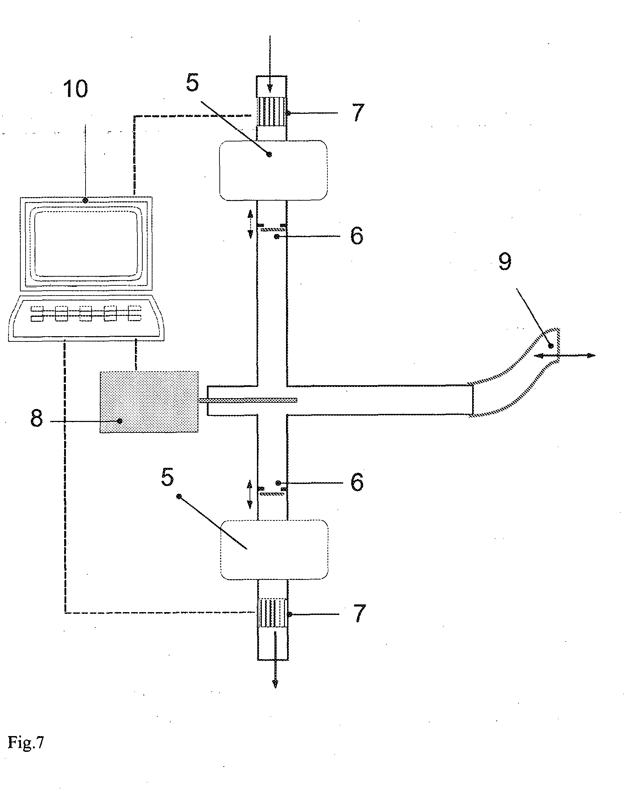

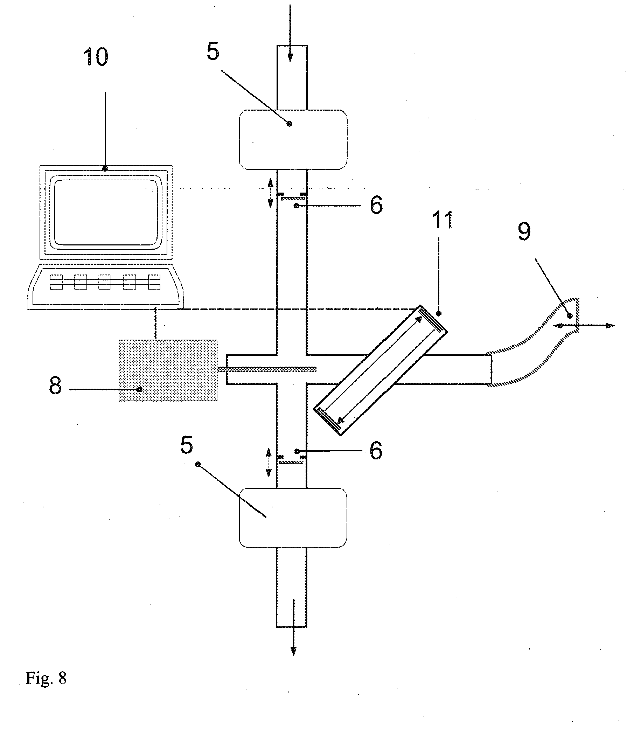

Bevorzugte Ausführungsformen der erfindungsgemäßen Vorrichtung sind in den Fig. 7 und 8 dargestellt und werden nachfolgend eingehender beschrieben.

Der Proband atmet über zwei Rückschlagventile, 6, durch ein T-Stück mit Mundstück, 9, ein und aus. Die Rückschlagventile sichern eine eindeutige Aufteilung der Atemluft in Inhalations- und Exhalationsvolumen. Sowohl die inhalierte als auch die exhalierte Luft wird mittels Absolutfilter, 5, vollständig von Partikeln gereinigt. Der Atemstrom kann entweder durch zwei Pneumotachographen, 7, im Inhalations- und Exhalationszweig oder aber über ein Ultraschallspirometer, 11, welches unmittelbar hinter dem Mundstück angebracht werden kann, gemessen werden. Die Messung der Partikelkonzentration erfolgt in einem Teilstrom der Atemluft mittels eines so genannten Kondensationskernzählers, 8. Dieses Messverfahren erlaubt im Gegensatz zu reinen Streulichtpartikelzählern auch die Detektion von Partikeln mit Durchmessern kleiner als 0,1 µm. Solche Partikel werden wegen ihres geringen Streuchlichtsignals mit reinen Streulichtzählern nicht mehr erfasst. Im Kondensationskernzähler lässt man die submikronen Partikeln durch Aufkondensieren eines übersättigten Dampfes (Butanol, Isopropanol, Wasser) auf Größen oberhalb von einigen Mikrometern anwachsen. Diese Partikeln können dann in einer einfachen Streulichtzelle gezählt werden. Aus der Zählrate, *R_{N}*, und dem Probenahmevolumenstrom ergibt sich die Konzentration, *C_{N}* der Partikel im Exhalat. Es ist vorteilhaft die Atemmanöver so durchzuführen, dass der Atemvolumenstrom immer größer ist als der Probenahmevolumenstrom. Dann ergibt sich die pro Atemzug emittierte Partikelanzahl durch einfaches Aufsummieren aller Zählereignisse während der Exhalationshalbwelle und Multiplikation mit dem Verhältnis aus Atemvolumen und Probenvolumen. Mit einem PC 10 werden der Messvorgang gesteuert, die anfallenden Daten aufgezeichnet und die notwendigen Berechnungen durchgeführt. Er kann überdies über den Bildschirm dem Probanden visuell Vorgaben für die Durchführung des Atemmanövers geben.

Kondensationskernzähler sind kommerziell erhältlich. In vielen Geräten ist aber der Probenahmevolumenstrom mit 0,11/min so gering, dass bei den insgesamt niedrigen Emissionsraten eine Erfassung der Zählereignisse nur mit geringerer statistischer Sicherheit erfolgen kann. Weiterhin werden in den Geräten zum Teil gesundheitsschädliche Dämpfe (beispielsweise Butanol) verwendet, die anschließend aufwendig gefiltert werden müssen. Überdiese sind kommerzielle Geräte nicht zuletzt wegen ihres hohen technischen Aufwands zur Sättigung des Dampfes und einer anschließenden kontrollierten Abkühlung teuer.

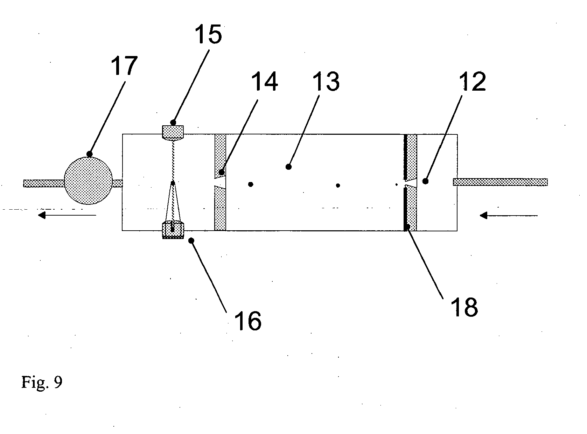

In der erfindungsgemäßen Vorrichtung wird die Wasserdampfkondensation aus der schon mit Wasserdampf gesättigten Atemluft ausgenutzt. In diesem Verfahren wird nach Fig. 8 der Probenahmevolumenstrom, charakterisiert durch eine Temperatur zwischen 35 und 37°C und einer relativen Wasserdampffeuchte von nahezu 100%, über eine Düse adiabatisch expandiert. Die mit der adiabatischen Expansion verbundene Abkühlung führt zu einer Übersättigung des Wasserdampfes und dessen Kondensation auf die in der Probenluft vorhandenen Partikeln. Nach Durchlaufen einer genügend langen Wachstumsstrecke werden die Partikel durch eine einfache Streulichtoptik detektiert und gezählt. Ein Ausführungsbeispiel dieses Prinzips zeigt die Fig. 9.

Das angesaugte Exhalat (T=37°C=310K, r.F.=100%) wird mit einem Volumenstrom von 1,4 1/min (0,4-21/min) angesaugt und in einer konvergierenden Düse, 12, (d=0,5 mm, (0,2-1mm) adiabatisch expandiert. Das Expansionsverhältnis *P1lP2* beträgt 1,11. (*P1*: Druck vor der Düse, *P2*: Druck hinter der Düse). Mit Hilfe der Zustandsgleichung für die adiabatische Expansion errechnet sich ein Temperaturabfall von 37°C auf 28°C, verbunden mit einer Reduktion des Sättigungsdampfdrucks des Wasserdampfs von 6,28 kPa auf 3,70 kPa, was einem Sättigungsverhältnis von *S_{V}*= 1,70 entspricht. Es findet keine Wiedererwärmung des Atemgases statt, weil die Außentemperatur in der Regel unterhalb von 28°C liegt. Unter diesen Bedingungen kondensiert Wasserdampf auf Partikeln mit Durchmessern größer als 0,05 µm. Diese wachsen innerhalb der Wachstumstrecke, 13 an und werden in einer zweiten, größeren Düse, 14 fokussiert. Sie durchlaufen ein durch den optischen Strahlengang mit Hilfe einer Zylinderlinse definiertes flaches Messvolumen. Der primäre Lichtstrahl der Sendeoptik, 15, wird in einer Lichtfalle der Empfangsoptik , 16, absorbiert. Das in einem engen Winkelbereich in Vorwärtsrichtung gestreute Licht, wird von der Empfangsoptik, 16, auf einen Photodetektor geleitet. Die Impulse werden verstärkt und vom PC erfasst. Über eine Pumpe, 17 wird der konstante Probenahmevolumenstrom eingestellt. Um Wasserdampfkondensation in der Düse zu vermeiden, besteht der Düsenträger aus thermisch gut leitendem Material und wird mit einem Peltierelement, 18, geheizt. Wasserdampfkondensation an den Wänden der Wachstumsstrecke ist für die Messung ohne Bedeutung.

## Patentansprüche

1. Eine Vorrichtung zur Analyse von Atemluft, umfassend
- ein Rohr zur Aufnahme von Atemluft,
- zumindest ein Spirometer, und
- einen Kondensationskernzähler,
**dadurch gekennzeichnet, dass**
der Kondensationskernzähler keine Befeuchtungszone aufweist.

2. Die Vorrichtung nach Anspruch 1, wobei das Rohr einen Inhalationszweig, der ausschließlich die Aufnahme von Atemluft aus der Umgebung ermöglicht, und einen Exhalationszweig, der ausschließlich die Abgabe von Atemluft an die Umgebung ermöglicht, aufweist, wobei bevorzugt sowohl im Inhalationszweig als auch im Exhalationszweig des Rohres ein Rückschlagventil angebracht ist.

3. Die Vorrichtung nach Anspruch 1 oder 2, wobei der Spirometer aus einem Pneumotachographen, einem Ultraschallspirometer, oder Kombinationen davon ausgewählt wird.

4. Die Vorrichtung nach Anspruch 2 oder 3, wobei im Inhalationszweig des Rohres, bevorzugt auch im Exhalationszweig des Rohres ein Filter, bevorzugt ein Absolutfilter angebracht ist.

5. Die Vorrichtung nach einem der vorstehenden Ansprüche, wobei der Kondensationskernzähler eine Düse mit in Strömungsrichtung konvergierendem Düsenquerschnitt für eine adiabatische Expansion aufweist.

6. Die Vorrichtung nach einem der vorstehenden Ansprüche, wobei der Kondensationskernzähler eine Streulichtoptik für die Detektion von Partikeln aufweist, welche bevorzugt eine Sendeoptik zur Erzeugung eines Primärlichtstrahls und eine Empfangsoptik mit Lichtfalle beinhaltet.

7. Die Vorrichtung nach Anspruch 5 oder 6, wobei die Düse für die adiabatische Expansion in einem Düsenträger angebracht ist, der bevorzugt aus einem Material mit guter thermischer Leitfähigkeit, bevorzugt Kupfer und/oder Messing, gefertigt ist und optional mit einem Heizelement in thermischem Kontakt steht.

8. Die Vorrichtung nach einem der vorstehenden Ansprüche zur nicht-invasiven Diagnostik von krankhaften Veränderungen der Lunge, insbesondere der peripheren Bereiche der Lunge.

9. Ein Verfahren zur Analyse von Atemluft, umfassend
(a) optional die Messung mindestens eines Lungenfunktionsparameters LFP, welcher bevorzugt ausgewählt wird aus der Lungenkapazität TLC, dem zur Atmung verfügbaren Lungenvolumen VC, dem nicht ventilierbaren Residualvolumen RV, dem Atemzugvolumen in Ruheatmung VT, dem Lungenvolumen bei Atemruhelage nach normaler Exspiration FRC, dem Lungenvolumen am Ende des Exspirationsvorgangs EELV, oder Kombinationen davon, an einem Probanden P₁ durch Spirometrie und/oder Bodyplethysmographie,
(b) die gleichzeitige Messung des Atemflusses und der Konzentration Cₑ von Partikeln in der Ausatemluft für n unterschiedliche durchschnittliche Ausatemvolumina EVₓ, wobei n≥1 ist und x von 1 bis n läuft, an dem Probanden P₁
(c) optional die Bestimmung der durchschnittlichen Anzahl Nₑ der Partikel in der Ausatemluft für jedes der unterschiedlichen durchschnittlichen Ausatemvolumina EV_{x,}
(d) die Bestimmung einer Funktion Cₑ = f(EVₓ/A) und/oder einer Funktion Nₑ = f(EVₓ/A), wobei A entweder 1 oder einer der im optionalen Schritt (a) ermittelten Lungenfunktionsparameter LFP ist,
(e) die Bestimmung einer Partikelemissionskennzahl EKZ für den Probanden P₁ durch Auswahl eines definierten Funktionswertes oder Funktionsarguments der Funktion Cₑ = f(EVₓ/A) bzw. Nₑ = f(EVₓ/A), wobei A die in Schritt (d) angegebene Bedeutung hat,
(f) die Wiederholung der Verfahrensschritte (a)-(d) an n Probanden Pₓ, wobei n≥1 ist und x von 2 bis n+1 läuft, und alle Probanden Pₓ sich untereinander und gegenüber P₁ durch mindestens einen biologischmedizinischen Parameter BMP unterscheiden,
(g) die Bestimmung der Partikelemmissionskennzahl EKZ für jeden Probanden Pₓ,
(h) die Bestimmung einer Funktion EKZ = f(BMP);
**dadurch gekennzeichnet, dass**
die Messung der Konzentration von Partikeln in der Ausatemluft mit einem Kondensationskernzähler erfolgt und der Kondensationskernzähler keine Befeuchtungszone aufweist.

10. Das Verfahren nach Anspruch 9, wobei die Messung des Atemflusses für unterschiedliche durchschnittliche Ausatemvolumina EVₓ in Schritt (b) durch Spirometrie, bevorzugt mit einem Ultraschallspirometer, einem Pneumotachographen oder Kombinationen davon, und/oder durch Bodyplethysmographie erfolgt.

11. Das Verfahren nach einem der Ansprüche 9-10, wobei der biologischmedizinische Parameter, durch den sich die Probanden voneinander unterscheiden, deren Alter ist.

12. Das Verfahren nach einem der Ansprüche 9-11, wobei die Schritte (c)-(e), (g) und (h) jeweils unter Verwendung einer Recheneinheit durchgeführt werden, wobei es sich bevorzugt um dieselbe Recheneinheit handelt, und wobei bevorzugt die in Schritt (b) erhaltenen Messdaten in einem Datenspeicher abgelegt werden und die Recheneinheit bzw. Recheneinheiten programmtechnisch so eingerichtet ist bzw. sind, dass anhand der Messdaten aus Schritt (b) die Bestimmung der durchschnittlichen Anzahl Nₑ der Partikel in der Ausatemluft im optionalen Schritt (c), die Bestimmung einer Funktion Cₑ = f(EVₓ/A) bzw. Nₑ = f(EVₓ/A) in Schritt (d), die Bestimmung einer Partikelemissionskennzahl EKZ in den Schritten (e) und (g), und/oder die Bestimmung einer Funktion EKZ = f(BMP) in Schritt (h) erfolgen kann bzw. erfolgt.

13. Das Verfahren nach einem der Ansprüche 9-12, wobei das Verfahren unter Verwendung der Vorrichtung gemäß einem der Ansprüche 1-8 durchgeführt wird.

## Claims

1. Device for analysing respiratory air, comprising
- a tube for receiving respiratory air,
- at least one spirometer and
- a condensation nuclei counter,
**characterized in that**
the condensation nuclei counter does not have a moistening zone.

2. Device according to Claim 1, wherein the tube has an inhalation branch, which exclusively enables the reception of respiratory air from the surroundings, and an exhalation branch, which exclusively enables the emission of respiratory air to the surroundings, wherein a check valve is preferably arranged both in the inhalation branch and in the exhalation branch of the tube.

3. Device according to Claim 1 or 2, wherein the spirometer is selected from a pneumotachograph, an ultrasonic spirometer or combinations thereof.

4. Device according to Claim 2 or 3, wherein a filter, preferably an absolute filter, is attached to the inhalation branch of the tube and preferably also to the exhalation branch of the tube.

5. Device according to one of the preceding claims, wherein the condensation nuclei counter has a nozzle, with a nozzle cross section converging in the flow direction, for adiabatic expansion.

6. Device according to one of preceding claims, wherein the condensation nuclei counter has a stray light optical unit for detecting particles, which optical unit preferably contains a transmission optical unit for generating a primary light beam and a reception optical unit with a light trap.

7. Device according to Claim 5 or 6, wherein the nozzle for the adiabatic expansion is attached to a nozzle support, which preferably is manufactured from a material with good thermal conductivity, preferably copper and/or brass, and is optionally in thermal contact with a heating element.

8. Device according to one of the preceding claims, for non-invasive diagnostics of pathological changes in the lung, in particular in the peripheral regions of the lung.

9. Method for analysing respiratory air, comprising
(a) optionally measuring at least one lung function parameter LFP, which is preferably selected from the total lung capacity TLC, vital capacity VC, the residual volume RV, the tidal volume VT, the functional residual capacity FRC, the end-expiratory lung volume EELV or combinations thereof on a subject P₁ by spirometry and/or body plethysmography,
(b) simultaneously measuring the respiratory flow and the concentration Cₑ of particles in the expired air for n different mean expired volumes EVₓ, where n ≥ 1 and x runs from 1 to n, on the subject P₁,
(c) optionally determining the mean number Nₑ of particles in the expired air for each of the different mean expired volumes EVₓ,
(d) determining a function Cₑ = f (EVₓ/A) and/or a function Nₑ = f(EVₓ/A), where A either equals 1 or is one of the lung function parameters LFP established in optional step (a),
(e) determining a particle emission characteristic EKZ for the subject P₁ by selecting a defined functional value or functional argument of the function Cₑ = f (EVₓ/A) or Nₑ = f (EVₓ/A), where A has the meaning defined in step (d),
(f) repeating method steps (a)-(d) on n subjects Pₓ, where n ≥ 1 and x runs from 2 to n+1, and all subjects Pₓ differ from one another and with respect to P₁ by at least one biological/medical parameter BMP,
(g) determining the particle emission characteristic EKZ for each subject Pₓ,
(h) determining a function EKZ = f(BMP);
**characterized in that**
the concentration of particles in the expired air is measured by means of a condensation nuclei counter and the condensation nuclei counter does not have a moistening zone.

10. Method according to Claim 9, wherein the measurement of the respiratory flow for different mean expired volumes EVₓ in step (b) is carried out by spirometry, preferably using an ultrasonic spirometer, a pneumotachograph or combinations thereof, and/or by body plethysmography.

11. Method according to either of Claims 9 and 10, wherein the biological/medical parameter by means of which the subjects differ from one another is the age thereof.

12. Method according to one of Claims 9-11, wherein steps (c)-(e), (g) and (h) are carried out in each case using a computer, wherein this preferably is the same computer and wherein, preferably, the measurement data obtained in step (b) are stored in a data storage medium and the computer or computers are configured in such a way by program-technical means that the measurement data from step (b) can be or is used to determine the mean number Nₑ of particles in the expired air in optional step (c), determine a function Cₑ = f (EVₓ/A) or Nₑ = f (EVₓ/A) in step (d), determine a particle emission characteristic EKZ in steps (e) and (g) and/or determine a function EKZ = f(BMP) in step (h).

13. Method according to one of Claims 9-12, wherein the method is carried out using the device according to one of Claims 1-8.

## Revendications

1. Dispositif d'analyse d'air respiratoire, comprenant
- un tube pour la prise d'air respiratoire,
- au moins un spiromètre, et
- un compteur de noyaux de condensation,
**caractérisé en ce que**
le compteur de noyaux de condensation ne présente pas de zone d'humidification.

2. Le dispositif selon la revendication 1, dans lequel le tube présente une branche d'inhalation qui permet exclusivement la prise d'air respiratoire à partir du milieu ambiant, et une branche d'exhalation qui permet exclusivement la remise d'air respiratoire au milieu ambiant, une vanne anti-retour étant de préférence disposée aussi bien dans la branche d'inhalation que dans la branche d'exhalation du tube.

3. Le dispositif selon la revendication 1 ou 2, dans lequel le spiromètre est sélectionné parmi un pneumotachographe, un spiromètre à ultrasons, ou des combinaisons de ceux-ci.

4. Le dispositif selon la revendication 2 ou 3, dans lequel un filtre, de préférence un filtre absolu, est disposé dans la branche d'inhalation du tube, de préférence également dans la branche d'exhalation du tube.

5. Le dispositif selon l'une des revendications précédentes, dans lequel le compteur de noyaux de condensation présente une tuyère dont la section transversale de tuyère converge en sens d'écoulement pour une expansion adiabatique.

6. Le dispositif selon l'une des revendications précédentes, dans lequel le compteur de noyaux de condensation présente une optique à lumière dispersée pour la détection de particules, laquelle contient de préférence une optique émettrice destinée à générer un rayon lumineux primaire et une optique réceptrice à piège à lumière.

7. Le dispositif selon la revendication 5 ou 6, dans lequel la tuyère pour l'expansion adiabatique est disposée dans un porte-tuyère qui est fabriqué de préférence à partir d'un matériau de bonne conductibilité thermique, de préférence le cuivre et/ou le laiton, et est facultativement en contact thermique avec un élément chauffant.

8. Le dispositif selon l'une des revendications précédentes pour le diagnostic non invasif de modifications pathologiques du poumon, en particulier des zones périphériques du poumon.

9. Procédé d'analyse d'air respiratoire, comprenant
(a) facultativement la mesure d'au moins un paramètre de fonction pulmonaire LFP, lequel est sélectionné de préférence parmi la capacité pulmonaire TLC, le volume pulmonaire disponible pour la respiration VC, le volume résiduel non ventilable RV, le volume de souffle en respiration au repos VT, le volume pulmonaire en position de repos respiratoire après expiration normale FRC, le volume respiratoire à la fin du processus d'expiration EELV, ou des combinaisons de ceux-ci, sur un sujet P₁ par spirométrie et/ou bodypléthysmographie,
(b) la mesure simultanée du flux respiratoire et de la concentration Ce de particules dans l'air expiré pour n différents volumes expirés moyens EVₓ, sachant que n≥1 et que x va de 1 à n, chez le sujet P₁,
(c) facultativement la détermination du nombre moyen Ne de particules dans l'air expiré pour chacun des différents volumes expirés moyens EVₓ,
(d) la détermination d'une fonction Cₑ = f(EVₓ/A) et/ou d'une fonction Nₑ = f(EVₓ/A), sachant que A est soit 1 soit un des paramètres de fonction pulmonaire LFP déterminés à l'étape facultative (a),
(e) la détermination d'un coefficient d'émission de particules EKZ pour le sujet P₁ par sélection d'une valeur de fonction définie ou d'un argument de fonction défini de la fonction Cₑ = f(EVₓ/A) et/ou Nₑ = f(EVₓ/A), sachant que A a la signification indiquée à l'étape (d),
(f) la répétition des étapes de procédé (a) à (d) sur n sujets Pₓ, sachant que n≥1 et que x va de 2 à n+1, et tous les sujets Pₓ se distinguent les uns des autres et par rapport à P₁ par au moins un paramètre biologico-médical BMP,
(g) la détermination du coefficient d'émission de particules EKZ pour chaque sujet Pₓ,
(h) la détermination d'une fonction EKZ = f(BMP) ;
**caractérisé en ce que**
la mesure de la concentration de particules dans l'air expiré est effectuée avec un compteur de noyaux de condensation et le compteur de noyaux de condensation ne présente pas de zone d'humidification.

10. Le procédé selon la revendication 9, dans lequel la mesure du flux respiratoire pour différents volumes expirés moyens EVₓ à l'étape (b) est effectuée par spirométrie, de préférence avec un spiromètre à ultrasons, un pneumotachographe ou des combinaisons de ceux-ci, et/ou par bodypléthysmographie.

11. Le procédé selon l'une des revendications 9-10, dans lequel le paramètre biologico-médical par lequel les sujets se distinguent les uns des autres est leur âge.

12. Le procédé selon l'une des revendications 9 à 11, dans lequel les étapes (c) à (e), (g) et (h) sont effectuées chaque fois en utilisant une unité de calcul, sachant qu'il s'agit de préférence de la même unité de calcul, et sachant que les données de mesure obtenues à l'étape (b) sont de préférence stockées dans une mémoire de données et que l'unité de calcul / les unités de calcul est/sont configurée/s du point de vue du programme de telle sorte que la détermination du nombre moyen Nₑ de particules dans l'air expiré à l'étape facultative (c), la détermination d'une fonction Cₑ = f(EVₓ/A) et/ou Nₑ = f(EVₓ/A) à l'étape (d), la détermination d'un coefficient d'émission de particules EKZ aux étapes (e) et (g), et/ou la détermination d'une fonction EKZ = f(BMP) à l'étape (h) peuvent être effectuées ou sont effectuées à l'aide des données de mesure provenant de l'étape (b).

13. Le procédé selon l'une des revendications 9 à 12, dans lequel le procédé est effectué moyennant le dispositif selon l'une des revendications 1 à 8.
